# DEMANDE DE BREVET EUROPEEN

(11) **EP 1 595 525 A1**
(43) Date de publication de la demande: **16.11.2005**
(21) Numéro de dépôt: 05290889.4
(22) Date de dépôt: 21.04.2005
(51) Int. Cl.: A61K 7/075

(54) **Composition contenant l'association d'un copolymère bloc exclusivement constitué de blocs hydrophiles et d'un agent antifongique particulier**

(30) Priorité: 13.05.2004 FR 0450932
(71) Demandeur: L'OREAL, 75008 Paris (FR)
(72) Inventeur: Giroud, Franck, 92110 Clichy (FR); Panangatte, Lydia, 78300 Poissy (FR); Dubief, Claude, 78150 Le Chesnay (FR)
(74) Mandataire: Miszputen, Laurent

(57) **Abrégé**

L'invention se rapporte à une composition cosmétique destinée en particulier au traitement des pellicules et squames du cuir chevelu, comprenant au moins un agent antifongique choisi parmi les sels de pyridinethione, les dérivés de 1-hydroxy-2-pyrrolidone, le 2-2'-dithio-bis-(pyridine-N-oxide), les sulfures de sélénium et leurs mélanges, et au moins un copolymère bloc exclusivement constitué de blocs hydrophiles et comprenant au moins un bloc poly-ionique.

Elle se rapporte aussi à un procédé de traitement antipelliculaire, consistant à appliquer la composition ci-dessus sur le cuir chevelu puis à rincer ce dernier.

## Description

L'invention se rapporte à une composition cosmétique destinée en particulier au traitement des pellicules et squames du cuir chevelu, comprenant au moins un agent antifongique choisi parmi les sels de pyridinethione, les dérivés de 1-hydroxy-2-pyrrolidone, le 2-2'-dithio-bis-(pyridine-N-oxide), les sulfures de sélénium et leurs mélanges, et au moins un copolymère bloc exclusivement constitué de blocs hydrophiles et comprenant au moins un bloc poly-ionique.

Elle se rapporte aussi à un procédé de traitement antipelliculaire, consistant à appliquer la composition ci-dessus sur le cuir chevelu puis à rincer ce dernier.

Cette association possède des propriétés antifongiques améliorées et permet de lutter efficacement contre les pellicules et les squames du cuir chevelu. Elle est également efficace sur les cuirs chevelus sensibles et réactifs et confère, de plus, d'excellentes propriétés cosmétiques à la chevelure, tels qu'un toucher agréable, une brillance et une légèreté satisfaisantes.

Plus spécialement, l'invention a trait à un shampooing antipelliculaire.

En vue de combattre la formation de pellicules ou de squames sur le cuir chevelu, formation qui est généralement accompagnée d'une prolifération microbienne et/ou fongique, il est connu d'utiliser comme actifs anti-pelliculaires des produits inhibant la prolifération microbienne et/ou présentant des propriétés antifongiques comme les sels de pyridinethione, les dérivés de 1-hydroxy-2-pyrrolidone ou encore les sulfures de sélénium. Toutefois, ces actifs anti-pelliculaires présentent souvent une activité insuffisante, nécessitant l'application longue et répétée de ces produits pour que l'utilisateur commence à en sentir les bienfaits. Ceci entraîne souvent l'abandon précoce du traitement par les utilisateurs.

Il subsiste donc le besoin d'un produit antipelliculaire efficace, à action prolongée et rapide.

Il est connu du document WO-A-00/02449 de Rhodia des compositions aqueuses biocides pour le traitement biocide de surfaces kératiniques cutanées ou textiles, de surfaces dures industrielles ou domestiques. Ces compositions contiennent un agent biocide, un surfactant lorsque le biocide est hydrophobe, et un copolymère organique hydrosoluble ou hydrodispersible constitué d'au moins une unité macromoléculaire ou oligomère qui peut interagir avec le biocide ou les micelles du surfactant contenant ledit biocide si ce dernier est hydrophobe, et une unité macromoléculaire hydrophile qui peut interagir avec la surface à traiter et éventuellement avec le biocide.

Il est par ailleurs connu du document WO-A-98/56334 de l'Université du Nebraska une composition biologiquement active comprenant un complexe supramoléculaire constitué d'un copolymère à blocs, possédant au moins un segment non ionique hydrosoluble et au moins un segment poly-ionique, et d'un surfactant ionique possédant des groupes hydrophobes ; la charge ionique du surfactant est opposée à celle du segment poly-ionique du copolymère à blocs et les constituants du complexe sont liés par interaction entre les charges opposées et les groupes hydrophobes. En outre, lorsque ce complexe contient un surfactant anionique possédant une activité biologique, alors ce surfactant anionique possède une charge nette inférieure ou égale à 10. Cette composition peut, en outre, contenir des agents antifongiques tels que les azoles ou les macrolides ainsi que des composés comme l'acide sorbique, l'EthylèneDiamineTétraAcétique ou le chlorure de benzalkonium.

Il est, en outre connu du document WO-A-00/71591 de Rhodia une composition de lavage, en particulier ménager pour des objets gras tels que de la vaisselle et des plats utilisés pour faire la cuisine, contenant des copolymères blocs cationiques. A titre optionnel, cette composition peut contenir un grand nombre d'additifs dont des enzymes, des agents nacrants, des agents antifongiques, des parfums, des conservateurs, des silicones, des tensioactifs.

On connaît encore du document WO-A-03/050184 de Rhodia une composition cosmétique comprenant un copolymère dibloc A-B tel que : le bloc A est poly-cationique au pH de la composition ; le bloc B est neutre au pH de la composition et les blocs A et B contiennent tous deux des unités dérivées de monomère alpha-éthylénique insaturé. Ces copolymères sont destinés à améliorer le dépôt des autres ingrédients de la composition, comme les silicones ou les polymères cationiques, sur les cheveux.

Par ailleurs, il est connu du document WO-A-03/050185 de Rhodia une composition contenant : un polymère poly-ionique au pH de la composition, pouvant être poly-cationique ou poly-anionique ; un composé ionique au pH de la composition ; un copolymère comprenant au moins une première partie polyionique au pH de la composition, qui est poly-cationique si le composé ionique est anionique ou poly-anionique si le composé ionique est cationique, et une seconde partie neutre au pH de la formulation ; en outre, l'une au moins des ces parties est constituée d'unités dérivées de monomères mono alpha éthyléniques insaturés. Le composé ionique est préférentiellement un tensioactif.

On connaît encore des documents FR-A-2 840 205 et FR-A-2 840 206 de l'Oréal des compositions de lavage des cheveux, contenant, dans un milieu aqueux ou hydro-alcoolique cosmétiquement acceptable, un copolymère linéaire séquencé, anionique ou non ionique, comprenant un bloc hydrophobe et un bloc hydrophile, au moins un agent tensioactif anionique associé à un agent tensioactif non ionique et/ou un agent tensioactif amphotère et respectivement au moins un polymère cationique ou amphotère ou une silicone non-volatile à température ambiante. Ces compositions peuvent contenir à titre optionnel un grand nombre d'ingrédients cosmétiques dont des conservateurs, des agents antipelliculaires.

On connaît également dans le document WO003/050195 des compositions aqueuses pour le traitement capillaires contenant des copolymères blocs comprenant au moins un bloc poly-ionique et au moins un bloc neutre présentant un bonne affinité vis-à-vis du cheveu.

Aucun de ces documents de l'art antérieur n'évoque ou ne suggère le problème technique de renforcer l'activité de certains agents antifongiques contre les pellicules et les squames du cuir chevelu.

De façon surprenante, les inventeurs ont trouvé qu'il était possible de considérablement renforcer l'activité antipelliculaire de certains agents antifongiques en les associant à des copolymères blocs exclusivement constitués de blocs hydrophiles et comprenant au moins un bloc poly-ionique.

Aussi, l'invention a pour objet une composition contenant un milieu physiologiquement acceptable, au moins un agent antifongique, choisi parmi les sels de pyridinethione, les dérivés de 1-hydroxy-2-pyrrolidone, le 2-2'-dithio-bis-(pyridine-N-oxide), les sulfures de sélénium et leurs mélanges, et au moins un copolymère bloc exclusivement constitués de blocs hydrophiles et comprenant au moins un bloc poly-ionique.

L'invention a encore pour objet l'utilisation notamment cosmétique d'au moins un copolymère bloc exclusivement constitués de blocs hydrophiles et comprenant au moins un bloc poly-ionique comme agent pour renforcer l'activité antipelliculaire d'une composition notamment cosmétique contenant un milieu physiologiquement acceptable et au moins un agent antifongique choisi parmi les sels de pyridinethione, les dérivés de 1-hydroxy-2-pyrrolidone, le 2-2'-dithio-bis-(pyridine-N-oxide), les sulfures de sélénium et leurs mélanges.

L'invention a encore pour objet l'utilisation d'au moins un copolymère bloc exclusivement constitués de blocs hydrophiles et comprenant au moins un bloc poly-ionique pour la fabrication d'une composition antipelliculaire contenant un milieu physiologiquement acceptable et au moins un agent antifongique choisi parmi les sels de pyridinethione, les dérivés de 1-hydroxy-2-pyrrolidone, le 2-2'-dithio-bis-(pyridine-N-oxide), les sulfures de sélénium et leurs mélanges.

L'invention a encore pour objet un procédé cosmétique de traitement capillaire, comprenant l'application d'une composition cosmétique telle que définie précédemment sur les cheveux et/ou le cuir chevelu humains. Ce procédé de traitement présente les caractéristiques d'un procédé cosmétique dans la mesure où il permet d'améliorer l'esthétique des cheveux et du cuir chevelu en leur donnant un aspect amélioré. En outre, il peut être utilisé quotidiennement pendant plusieurs mois, sans prescription médicale.

Au sens de l'invention, "au moins un" signifie, un ou plusieurs ; au moins deux signifient deux ou plus ; etc.

Comme sel de pyridinethione utilisable dans l'invention, on peut citer les sels de calcium, de magnésium, de baryum, de strontium, de zinc, de zirconium. Le sel de zinc est particulièrement préféré.

Comme dérivé de 1-hydroxy-2-pyrrolidone utilisable dans l'invention, on peut citer le 1-hydroxy-4-methyl-6-(2,4,4,-trimethylpentyl)-2(1H)-pyridinone (ou piroctone), la piroctone olamine (ou octopirox).

Comme sulfure de sélénium utilisable dans l'invention, on peut citer le disulfure de sélénium, les polysulfures de sélénium de formule SeₓS_{y} dans laquelle x + y = 8.

En particulier, on utilise comme agent antifongique la pyridinethione de zinc, l'octopirox, le disufure de sélénium ou leurs mélanges.

Par "copolymère bloc", on entend un polymère résultant de la polymérisation d'au moins 2 monomères différents, chaque bloc comprenant au moins 3 motifs de répétition identiques, en général, au moins 10 motifs de répétition identiques.

Par « copolymère bloc exclusivement constitué de blocs hydrophiles », on entend par un copolymère bloc pour lequel tous les blocs sont hydrophiles.

Par "bloc hydrophile", on entend un bloc dont l'homopolymère correspondant est soluble ou dispersible dans l'eau à une quantité d'au moins 1 % en poids par rapport à l'ensemble (eau + homopolymère), à la température ambiante (20-25 °C) et pression atmosphérique (environ 10⁵ Pa). Cet homopolymère peut être introduit tel que dans l'eau ou être neutralisé. L'ensemble (eau + homopolymère) présente à l'oeil nu un aspect transparent ou translucide. En particulier, chaque bloc hydrophile comprend plus de 50 % en moles, par rapport au nombre total de moles du bloc, de monomère hydrophile.

Par "bloc poly-ionique", on entend un bloc comportant au moins 2 charges, ces charges pouvant être de même signe ou de signes opposés.

Selon l'invention, le ou les copolymères blocs utilisés comprennent au moins 2 blocs et par exemple 2 ou 3 blocs. Ce ou ces copolymères peuvent aussi être agencés en étoiles.

Selon l'invention, les copolymères à blocs hydrophiles polyioniques peuvent être choisis parmi : les copolymères à blocs linéaires ou branchés, hydrosolubles ou hydrodispersés, contenant a) au moins un bloc macromoléculaire polyionique et b) au moins un bloc macromoléculaire non ionique constitué majoritairement d'un ou plusieurs monomères hydrophiles ; les copolymères greffés.

Les copolymères à blocs hydrophiles poly-ioniques utilisables dans l'invention sont fabriqués selon les procédés connus de l'homme de l'art par copolymérisation séquencée d'au moins 2 monomères dont l'un au moins est choisi parmi les monomères hydrophiles ioniques comme les monomères hydrophiles anioniques, les monomères hydrophiles cationiques et/ou les monomères hydrophiles amphotères, et éventuellement d'au moins un monomère hydrophile non ionique. Les copolymères blocs de l'invention peuvent être préparés par les procédés de synthèse classiquement utilisés pour obtenir des polymères blocs. On peut citer par exemple les polymérisations anionique ou cationique, et la polymérisation radicalaire contrôlée (voir "New Method of Polymer Synthesis", Blackie Academic & Professional, Londres, 1995, volume 2, page 1, ou Trends Polym. Sci. 4, page 183 (1996) de C. J. Hawker), qui peut être mise en oeuvre suivant différents procédés comme par exemple la polymérisation radicalaire par transfert d'atomes (Atom Transfert Radical Polymerization ou ATRP) (voir JACS, 117, page 5614 (1995), de Matyjasezwski et al.), la méthode des radicaux tels que les nitroxydes (Georges et al., Macromolecules, 1993, 26, 2987).

La masse moléculaire moyenne en nombre de chaque bloc, qu'il soit, sous forme d'un copolymère ou d'un homopolymère, est de préférence comprise dans la gamme allant de 500 à 100 000, en particulier de 800 à 50 000 et mieux de 1500 à 40 000.

Parmi les monomères hydrophiles anioniques entrant dans la constitution des copolymères à blocs de l'invention, on peut citer notamment :
- les monomères à insaturation(s) éthylénique(s) comprenant au moins une fonction acide carboxylique, phosphorique ou sulfonique comme par exemple l'acide acrylique, l'acide méthacrylique, l'acide 1,4 phénylènediacrylique, l'acide crotonique, l'acide itaconique, l'acide fumarique, l'acide maléique, l'acide citraconique, l'acide diacrylique, l'acide coumarique, l'acide diméthylfumarique, l'acide mésaconique, l'acide muconique, l'acide acrylamidopropanesulfonique, l'acide 2-acrylamido 2-méthylpropanesulfonique, l'acide 4-styrène sulfonique, l'acide vinylbenzoïque, l'acide vinylphosphorique, l'acide vinyl benzène sulfonique, l'acide 2-propène-1-sulfonique, l'acide vinyl sulfonique, l'acide vinyl phosphonique, l'acide vinyl glycolique, l'acide éthylidènepropionique, l'acide éthylidènesuccinique, l'acide glutaconique, l'acide diallylmalonique, l'acide cinnamique et ses dérivés hydroxylé et/ou méthoxylé ;
- les anhydrides carboxyliques porteurs d'une liaison vinylique tels que l'anhydride maléique ou l'anhydride citraconique ;
- le (méth)acrylate de 2-carboxyéthyle, le méthacrylate ou l'acrylate de sulfopropyle (CH₂=C(CH₃)CO₂(CH₂)₃SO₃H), le maléate de diallyle (C₃H₅-CO₂-CH=CH-CO₂-C₃H₅), le méthacrylate ou l'acrylate de sulfoéthyle et la vinylméthylsulfone ;
- ainsi que leurs sels ;
- leurs associations.

En particulier, le monomère hydrophile anionique comporte une fonction carboxylique et est choisi parmi l'acide acrylique, l'acide 1,4 phénylènediacrylique, l'acide ou l'anhydride citraconique, l'acide cinnamique et ses dérivés hydroxylé et/ou méthoxylé, l'acide glutaconique, l'acide itaconique, l'acide méthacrylique, l'acide ou l'anhydride maléique, l'acide mésaconique, l'acide muconique ou hydromuconique.

La neutralisation des groupes anioniques peut être effectuée par une base minérale, telle que LiOH, NaOH, KOH, Ca(OH)₂, NH₄OH ou Zn(OH)₂ ; ou par une base organique telle qu'une alkylamine primaire, secondaire ou tertiaire, notamment la triéthylamine, la monoéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions hydroxyle ; on peut notamment citer l'amino-2-méthyl-2-propanol, la triéthanolamine et la diméthylamino-2-propanol. On peut encore citer la lysine ou la 3-(diméthylamino)propylamine.

Parmi les monomères hydrophiles cationiques entrant dans la constitution des copolymères à blocs de l'invention, on peut citer les monomères à insaturation(s) éthylénique(s) comprenant au moins une fonction amine primaire, secondaire ou tertiaire ; comme par exemple :
- la 2-vinylpyridine, la 4-vinylpyridine, les N- alkyle en C₁-C₆ de pipéridine ;
- les (méth)acrylates d'aminoalkyles tels que les (méth)acrylates de N,N-di(C₁-C₄) alkyle amino(C₁-C₆)alkyle ou les (méth)acrylates de (C₁-C₄)alkyle amino(C₁-C₆)alkyle et notamment le (méth)acrylate de N,N diméthylaminoéthyle, le (méth)acrylate de N,N diéthylaminoéthyle, le (méth)acrylate de 2-aminoéthyle, le (méth)acrylate de 2-(terbutylamino)éthyle ;
- les (méth)acrylamides d'aminoalkyles tels que les (méth)acrylamides de N,N-di(C₁-C₄)alkyle amino(C₁-C₆)alkyle ou les (méth)acrylamides de (C₁-C₄)alkyle amino(C₁-C₆)alkyle, et notamment le (méth)acrylamide de N,N diméthylaminopropyle, le (méth)acrylamide de N,N diméthylaminoéthyle, le (méth)acrylamide de 3-aminopropyle, l'acécaïnide ;
- la vinylamine, l'allylamine, la vinylimidazole, le 2-(diéthylamino)éthylstyrène, le (2-(diéthylamino)éthyl)-2(2-propényloxy)benzamide ;
- ainsi que leurs sels, leurs formes quaternisées ;
- leurs associations.

Parmi les formes salifiées des monomères cationiques, on peut citer les sels d'acides minéraux tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acides carboxyliques, sulfoniques ou phosphoniques. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique, l'acide oxalique et l'acide tartrique.

Les formes quatemisées des monomères cationiques peuvent être obtenues par réaction avec des composés à halogène mobile, comme notamment les halogénures d'alkyle tels que les chlorures ou les bromures d'alkyle en C₁-C₁₂, et par exemple le bromure de méthyle ou le chlorure d'éthyle ; ou encore avec le chloroacétate de sodium. Les formes quaternisées peuvent aussi être obtenues par réaction avec des sulfones cycliques, par exemple la propanesulfone.

Parmi les monomères hydrophiles amphotères entrant dans la constitution des copolymères à blocs de l'invention, on peut citer les monomères comportant au moins un atome d'azote basique et au moins un groupement carboxylique, phosphorique ou sulfonique comme notamment les monomères cationiques comportant une fonction amine primaire, secondaire, tertiaire ou quaternaire et une fonction carboxylique, phosphorique ou sulfonique ou bien les monomères zwittérioniques de carboxybétaïnes ou de sulfobétaïnes.

A titre d'exemple de monomère amphotère utilisable dans l'invention, on peut citer
- les monomères zwittérioniques de formule (I) suivante : 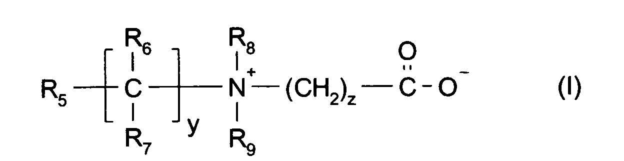
dans laquelle R₅ désigne un groupement insaturé polymérisable tel qu'un groupement acrylate, méthacrylate, acrylamide ou méthacrylamide, y et z représentent un nombre entier de 1 à 3, R₆ et R₇ représentent un atome d'hydrogène, méthyle, éthyle ou propyle, R₈ et R₉ représentent un atome d'hydrogène ou un radical alkyle de telle façon que la somme des atomes de carbone dans R₈ et R₉ ne dépasse pas 10 ;
- l'acide aminocinnamique, les acides aminés comme l'arginine ;
- les monomères répondant à la formule (II) suivante : 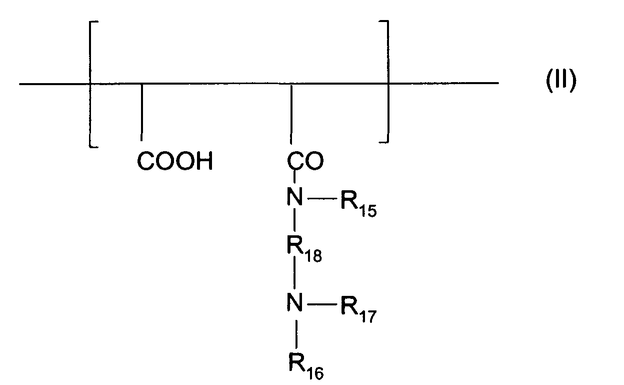
dans laquelle R₁₄ représente un atome d'hydrogène, un radical CH₃O, CH₃CH₂O, phényle, R₁₅ et R₁₆ désignent indépendamment l'hydrogène ou un radical alkyle inférieur en C₁ à C₆ tel que méthyle, éthyle, R₁₇ désigne un radical alkyle inférieur en C₁ à C₆ tel que méthyle, éthyle ou un radical répondant à la formule : -R₁₈-N(R₁₆)₂, R₁₈ représentant un groupement -CH₂-CH₂-, -CH₂-CH₂-CH₂-, -CH₂-CH(CH₃)-, R₁₆ ayant les significations mentionnées ci-dessus,
- l'anhydride maléique modifié partiellement par semiamidification avec une N,N-dialkylaminoalkylamine telle que la N,N-diméthylamino-propylamine ou par semiestérification avec une N,N-dialcanolamine ;
- leurs mélanges.

Parmi les monomères hydrophiles non ioniques entrant dans la constitution des copolymères à blocs de l'invention, on peut citer, entre autre, l'acrylamide, le méthacrylamide, les acrylamides N-alkylés en C₁₋₆ ou N,N-dialkylés en C₁₋₃, l'acrylate de polyéthylèneglycol, le méthacrylate de polyéthylèneglycol, le N-vinylacétamide, le N-méthyl-N-vinylacétamide, le N-vinylformamide, le N-méthyl-N-vinylformamide, les N-vinyllactames comportant un groupe cyclique de 4 à 9 atomes de carbone, l'alcool vinylique (copolymérisé sous forme d'acétate de vinyle puis hydrolysé), l'oxyde d'éthylène et/ou de propylène, l'acrylate d'hydroxyéthyle, la vinylpyrrolidone, le vinyle triazole, l'acrylate d'hydroxypropyle, le méthacrylate d'hydroxyéthyle, le méthacrylate d'hydroxypropyle, les acides aminés, le méthyle 2 ou éthyle 2 oxazoline.

Les copolymères utilisables dans l'invention sont en particulier ceux décrits dans les documents WO-A-03/050184, WO-A-03/050185 et WO03/050195 de Rhodia.

De préférence, on utilise les copolymères à blocs linéaires et en particulier les copolymères diblocs à blocs linéaires.

Plus préférentiellement, les copolymères blocs selon l'invention comprennent au moins un bloc hydrophile poly-ionique et au moins un bloc hydrophile non ionique.

Selon un mode particulier de mise oeuvre de l'invention, le ou les copolymères à blocs utilisables dans l'invention est choisi parmi les polymères de formule N-P, (P-N)ₙ-P, N-(P-N)ₙ, N-(P-N)ₙ-P, où N est un bloc hydrophile non-ionique, P est un bloc hydrophile polyionique et n est un entier allant de 1 à 5 000. Les séquences (P-N)ₙ de ces copolymères peuvent être de même longueur ou de longueur différente.

Selon un mode particulier de mise en oeuvre de l'invention, le copolymère de l'invention comprend un bloc hydrophile non ionique et un bloc hydrophile poly-cationique.

De façon avantageuse, le ou les copolymères à blocs utilisables dans l'invention comprennent un ou plusieurs blocs d'acide polyacrylique, d'acide poly(méth)acrylique ou de l'un de leurs sels ou de leurs amides et un ou plusieurs blocs de (méth)acrylates d'aminoalkyles ou de l'un de leurs sels ou de leur forme quaternisée. On peut citer comme exemple de ces blocs le bloc poly(acrylamide) et le bloc poly(méthosulfate de triméthylammonium éthylacrylate).

La composition selon l'invention peut contenir un ou plusieurs antifongiques cités précédemment. Leur quantité est fonction de la nature du milieu physiologique utilisé ainsi que de la quantité de copolymère à blocs hydrophiles utilisée et du niveau d'activité antipelliculaire recherché. En pratique, l'agent antifongique ou le mélange d'agents antifongiques peut représenter de 0,001% à 10% en poids par rapport au poids total de la composition et préférentiellement de 0,1 à 5% en poids.

La composition selon l'invention peut contenir un ou plusieurs copolymères à blocs cités précédemment. Leur quantité est aussi fonction de la nature du milieu physiologique utilisé ainsi que de la quantité d'agent antifongique utilisée et du niveau d'activité antipelliculaire recherché. En pratique, le copolymère à blocs ou le mélange de copolymères à blocs peut représenter de 0,01 à 20% en poids par rapport au poids total de la composition et préférentiellement de 0,05 à 10% en poids.

La composition selon l'invention contient, en outre, un milieu physiologiquement acceptable. Par "milieu physiologiquement acceptable", on entend un milieu non toxique et susceptible d'être appliqué sur le cuir chevelu et les cheveux d'êtres humains. Par "cosmétique", on entend au sens de l'invention une composition pouvant être vendue sans prescription médicale.

En particulier, ce milieu contient une phase aqueuse contenant de l'eau et éventuellement au moins un solvant organique miscible à l'eau comme les monoalcools en C₂ à C₆ tels que l'éthanol, l'isopropanol, le n-butanol, les polyols comme le propylèneglycol, le glycérol ou les éthers de glycol. Ce milieu peut contenir une phase huileuse contenant un ou plusieurs corps gras liquides à température ambiante (25°C) et pression atmosphérique, non miscibles à l'eau, appelées "huiles".

Le milieu physiologiquement acceptable peut, en outre, comprendre au moins un tensioactif choisi parmi les tensioactifs non-ioniques, les tensioactifs anioniques, les tensioactifs cationiques, les tensioactifs amphotères, et leurs mélanges.

La composition suivant l'invention, après application sur les cheveux et cuir chevelu humains peut être rincée ou non rincée à l'eau ou par un shampooing. Elle peut se présenter sous toute forme classiquement utilisée dans le domaine concerné et par exemple sous forme de lotion plus ou moins épaissie, de gel, de crème, de spray ou de mousse. Cette composition peut être monophasique ou multiphasique. Elle peut, en outre, se présenter par exemple sous forme d'un shampooing, d'un après shampooing, d'une lotion pour le traitement des cheveux et du cuir chevelu. Avantageusement, la composition de l'invention est sous forme d'un shampooing.

Aussi, l'invention a encore pour objet un shampooing pour cheveux, contenant un milieu physiologiquement acceptable, une base lavante, au moins un agent antifongique et au moins un copolymère bloc exclusivement constitué de blocs hydrophiles tels que définis précédemment.

La composition selon l'invention peut, en outre, contenir comme base lavante au moins un agent tensioactif choisi parmi les agents tensioactifs anioniques, non-ioniques, amphotères et leurs mélanges.

Comme agent tensioactif anionique utilisable dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylaryl-polyéthersulfates, les monoglycéride-sulfates ; les alkylsulfonates, les alkylamidesulfonates, les alkylaryl-sulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle, leurs mélanges.

On peut également utiliser comme agent tensioactif anionique, les monoesters d'alkyle en C₆-C₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone, leurs mélanges.

Un autre groupe d'agents tensioactifs anioniques utilisable dans la composition de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides alkyl(C₆₋₂₄)éther-carboxyliques polyoxyalkylénés, les acides alkyl(C₆₋₂₄)aryl(C₆₋₂₄)éther-carboxyliques poly-oxyalkylénés, les acides alkyl(C₆-₂₄)amidoéther-carboxyliques poly-oxyalkylénés et leurs sels, en particulier ceux comportant de 2 à 50 motifs oxyde d'éthylène, et leurs mélanges.

On utilise de préférence comme agent tensioactif anionique, les alkylsulfates, les alkyléthersulfates et les alkyléthercarboxylates, leurs mélanges, en particulier sous forme de sels de métaux alcalins ou alcalino-terreux, d'ammonium, d'amine ou d'aminoalcool.

Comme agent tensioactif amphotère utilisable dans la présente invention, on peut citer les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les alkyl(C₈₋₂₀)amidoalkyl(C₆₋₈)-bétaïnes, les alkyl(C₈₋₂₀)amidoalkyl(C₆₋₈)sulfo-bétaïnes et leurs mélanges.

Parmi les dérivés d'amines, on peut citer les produits commercialisés sous la dénomination MIRANOL®, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO⁻) (1)

dans laquelle :
. Rₐ représente un groupe alkyle dérivé d'un acide Rₐ-COOH présent dans l'huile de coprah hydrolysée, un groupe heptyle, nonyle ou undécyle,
. R_{b} représente un groupe bêta-hydroxyéthyle, et
. R_{c} représente un groupe carboxyméthyle ; et

   Rₐ'-CONHCH₂CH₂-N(B)(C) (2)
dans laquelle :
. B représente -CH₂CH₂OX',
. C représente -(CH₂)_{z}-Y', avec z = 1 ou 2,
. X' représente le groupe -CH₂CH₂-COOH ou un atome d'hydrogène,
. Y' représente -COOH ou le groupe -CH₂-CHOH-SO₃H,
. Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations coco-amphodiacétate de disodium, lauro-amphodiacétate de disodium, caprylamphodiacétate de disodium, caprylo-amphodiacétate de disodium, coco-ampho-dipropionate de disodium, lauro-ampho-dipropionate de disodium, capryl-ampho-dipropionate de disodium, caprylo-ampho-dipropionate de disodium, acide lauro-ampho-dipropionique, acide coco-ampho-dipropionique.

A titre d'exemple, on peut citer le cocoamphodiacétate commercialisé par la société RHODIA sous la dénomination commerciale MIRANOL® C2M concentré.

Parmi les agents tensioactifs amphotères, on utilise de préférence les (alkyle en C₈₋₂₀)-bétaïnes, les (alkyle en C₈₋₂₀)-amido(alkyle en C₆₋₈)bétaïnes, les alkylamphodiacétates et leurs mélanges.

Comme agent tensioactif non-ionique utilisable dans la composition selon l'invention, on peut citer les composés connus décrits notamment dans le livre "Handbook of Surfactants" par M.R. PORTER, éditions Blackie & Son (Glasgow and London), 1991, pp 116-178). Il est choisi notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30, leurs mélanges.

On peut également citer comme agent tensioactif non-ionique utilisable dans l'invention les condensats d'oxyde d'éthylène et d'oxyde de propylène sur des alcools gras ; les amides gras polyéthoxylés ayant de préférence de 2 à 30 motifs d'oxyde d'éthylène ; les amides gras polyglycérolés comportant en moyenne de 1 à 5 groupements glycérol et en particulier de 1,5 à 4 ; les esters d'acides gras du sorbitane éthoxylés ayant de 2 à 30 motifs d'oxyde d'éthylène ; les esters d'acides gras du saccharose ; les esters d'acides gras de polyéthylèneglycol ; les (alkyle en C₆₋₂₄)polyglycosides ; les dérivés de N-(alkyle en C₆₋₂₄)glucamine ; les oxydes d'amines tels que les oxydes d'(alkyle en C₁₀₋₁₄)amines ou les oxydes de N-(acyle en C₁₀₋₁₄)-aminopropylmorpholine ; leurs mélanges.

Parmi les tensioactifs non-ioniques cités ci-dessus, on utilise de préférence les (alkyle en C₆₋₂₄)polyglycosides.

Selon un mode de réalisation particulier de l'invention, la base lavante contient au moins un tensioactif anionique et au moins un tensioactif amphotère ou non-ionique.

La quantité totale d'agent tensioactif est généralement comprise dans la gamme allant de 0,01 % à 50% en poids, de préférence de 0,1 % à 25 % en poids par rapport au poids total de la composition.

En particulier, lorsque la composition de l'invention se présente sous forme d'un shampooing, la quantité totale d'agents tensioactifs (ou base lavante) est en particulier choisie de 4 % à 50 % en poids, par exemple de 8 % à 25 % en poids, rapportée au poids total de la composition cosmétique.

La composition selon l'invention peut, en outre, contenir au moins un ingrédient additionnel utilisé classiquement dans les domaines considérés et choisi parmi des principes actifs cosmétiques ayant un effet bénéfique sur la peau et/ou sur les cheveux comme les sels de zinc d'acide organique (acétate, glycolate, lactate, gluconate ou citrate) ou minéral (chlorure et le sulfate), les vitamines (E, C, B₂, B₅, F), les filtres UV, les agents anti-radicalaires, les conservateurs, les céramides, les extraits de végétaux et des additifs de formulation tels que des polymères filmogènes anioniques, non ioniques, cationiques ou amphotères, différents des copolymère à blocs hydrophiles polyioniques décrits ci-dessus, des épaississants polymériques de phase aqueuse ou de phase huileuse, des épaississants non-polymériques de phase aqueuse comme des sels ou des amides d'acide gras hydroxylés ou non, des agents nacrants, des agents opacifiants, des colorants solubles dans le milieu, des pigments, des charges, des parfums, des huiles d'origine minérale, végétale et/ou synthétique, des esters d'acides et/ou d'alcools gras, des cires, des agents de stabilisation de pH comme les acides, les bases, les sels, des solvants organiques, des silicones, des électrolytes, et leurs mélanges.

Les quantités des différents ingrédients additionnels de la composition selon l'invention sont celles généralement utilisées dans les domaines considérés et sont en particulier comprises dans la gamme allant de 0,001 à 20% du poids total de la composition. En outre, cette composition est préparée selon les méthodes usuelles.

Bien entendu l'homme du métier veillera à choisir les éventuels ingrédients additionnels et/ou leur quantité de telle manière que les propriétés avantageuses de la composition selon l'invention, à savoir l'activité antipelliculaire ne soit pas ou substantiellement pas, altérée par l'adjonction envisagée.

Avantageusement, le pH de la composition de la présente invention est choisi dans la gamme allant de 2 à 11 et préférentiellement de 3 à 10 par exemple de 5 à 8.

### EXEMPLES

On va maintenant donner à titre d'illustration des exemples de réalisation de l'invention qui ne sauraient limiter en aucune façon sa portée. Les concentrations des différents ingrédients de la composition sont données en pourcentage massique.

### Exemples 1 et 2 :

On a déterminé l'efficacité de deux antifongiques, le zinc pyridinethione (omadine de Zn fabriquée ou vendue par la société Olin) et la piroctone olamine (octopirox fabriqué ou vendu par la société Hoechst) à la concentration de 1 % en Matière Active (M.A.) dans une base lavante tensioactive constituée d'un lauryléther (2,2 moles) sulfate de sodium à 10% M.A. et de cocopropylbétaïne à 5% M.A. en présence ou non de 1% M.A d'un copolymère dibloc poly(méthosulfate de triméthylammonium éthylacrylate)-b-poly(acrylamide) (de poids moléculaire moyenne en poids de 11000/30000. Le reste de la composition contient une quantité suffisante d'eau pour avoir 100 %.

Le test utilisé pour comparer l'efficacité des compositions selon l'invention par rapport à des compositions exemptes de copolymère à blocs hydrophiles est la méthode classique dite de Concentration Minimale Inhibitrice (CMI) notamment décrite dans le document EP-A-1 048 288.

De façon très nette la présence du copolymère dibloc améliore l'efficacité antipelliculaire des antifongiques considérés.

## Revendications

1. Composition contenant un milieu physiologiquement acceptable, au moins un agent antifongique choisi parmi les sels de pyridinethione, les dérivés de 1-hydroxy-2-pyrrolidone, le 2-2'-dithio-bis-(pyridine-N-oxide), les sulfures de sélénium et leurs mélanges, et au moins un copolymère bloc exclusivement constitué de blocs hydrophiles et comprenant au moins un bloc poly-ionique.

2. Composition selon la revendication 1, **caractérisée en ce que** les sels de pyridinethione sont les sels de calcium, de magnésium, de baryum, de strontium, de zinc, de zirconium.

3. Composition selon la revendication 1, **caractérisée en ce que** le dérivé de 1-hydroxy-2-pyrrolidone est choisi parmi la piroctone ou la piroctone olamine.

4. Composition selon l'une des revendications précédentes, **caractérisée en ce que** l'agent antifongique est choisi parmi la pyridinethione de zinc, l'octopirox, le disufure de sélénium et leurs mélanges.

5. Composition selon l'une des revendications précédentes, **caractérisée en ce que** le copolymère est un copolymère à blocs linéaires.

6. Composition selon l'une des revendications précédentes, **caractérisée en ce que** le copolymère est un copolymère dibloc à blocs linéaires.

7. Composition selon l'une des revendications précédentes, **caractérisée en ce que** chaque bloc du copolymère présente une masse moléculaire moyenne en nombre de 500 à 100 000, en particulier de 800 à 50 000.

8. Composition selon l'une des revendications précédentes, **caractérisée en ce que** le copolymère comprend au moins un bloc hydrophile non-ionique et au moins un bloc hydrophile poly-ionique.

9. Composition selon l'une des revendications précédentes, **caractérisée en ce que** le copolymère est choisi parmi les polymères de formule N-P, (P-N)ₙ-P, N-(P-N)ₙ, N-(P-N)ₙ-P, où N est un bloc hydrophile non ionique, P est un bloc hydrophile poly-ionique et n est un entier allant de 1 à 5 000.

10. Composition selon l'une des revendications précédentes, **caractérisée en ce que** le copolymère comprend un bloc hydrophile non ionique et un bloc hydrophile poly-cationique.

11. Composition selon l'une des revendications précédentes, **caractérisée en ce que** le copolymère comprend un ou plusieurs blocs d'acide polyacrylique, d'acide poly(méth)acrylique ou de l'un de leurs sels ou de leurs amides et un ou plusieurs blocs de (méth)acrylate d'aminoalkyle ou de l'un de leurs sels ou de leur forme quaternisée.

12. Composition selon l'une des revendications précédentes, **caractérisée en ce que** l'agent antifongique ou le mélange d'agents antifongiques représente de 0,001% à 10% en poids par rapport au poids total de la composition et préférentiellement de 0,1 à 5% en poids.

13. Composition selon l'une des revendications précédentes, **caractérisée en ce que** le copolymère à blocs ou le mélanges de copolymères à blocs représente de 0,01 à 20% en poids par rapport au poids total de la composition et préférentiellement de 0,05 à 10% en poids.

14. Composition selon l'une des revendications précédentes, **caractérisée en ce que** le milieu contient de l'eau et éventuellement au moins un solvant organique miscible à l'eau.

15. Composition selon l'une des revendications précédentes, **caractérisée en ce que** la composition se présente sous forme de lotion plus ou moins épaissie, de gel, de crème, de spray ou de mousse.

16. Composition selon l'une des revendications précédentes, **caractérisée en ce que** la composition est sous forme monophasique ou multiphasique.

17. Composition selon l'une des revendications précédentes, **caractérisée en ce que** le milieu physiologiquement acceptable comprend au moins un tensioactif choisi parmi les tensioactifs non-ioniques, les tensioactifs anioniques, les tensioactifs cationiques, les tensioactifs amphotères, et leurs mélanges.

18. Composition selon revendication précédente, **caractérisée en ce que** la quantité totale d'agent tensioactif est comprise dans la gamme allant de 0,01 % à 50% en poids, de préférence de 0,1 % à 25 % en poids par rapport au poids total de la composition.

19. Composition selon l'une des revendications précédentes, **caractérisée en ce que** la composition contient une base lavante.

20. Composition selon la revendication précédente, **caractérisée en ce que** la base lavante contient au moins un agent tensioactif choisi parmi les agents tensioactifs anioniques, non-ioniques, amphotères et leurs mélanges.

21. Composition selon l'une des revendications 19 ou 20, **caractérisée en ce que** la base lavante contient au moins un tensioactif anionique et au moins un tensioactif amphotère ou non-ionique.

22. Composition selon l'une des revendications 19 à 21, **caractérisée en ce que** la base lavante représente de 4 % à 50% en poids, de préférence de 8 % à 25 % en poids par rapport au poids total de la composition.

23. Composition selon l'une des revendications précédentes, **caractérisée en ce que** la composition contient au moins un ingrédient additionnel choisi parmi les principes actifs cosmétiques et les additifs de formulation.

24. Shampooing pour cheveux contenant un milieu physiologiquement acceptable, une base lavante, au moins un agent antifongique choisi parmi les sels de pyridinethione, les dérivés de 1-hydroxy-2-pyrrolidone, le 2-2'-dithio-bis-(pyridine-N-oxide), les sulfures de sélénium et leurs mélanges et un copolymère bloc exclusivement constitué de blocs hydrophiles tel que défini dans l'une quelconque des revendications précédentes.

25. Procédé cosmétique de traitement capillaire, comprenant l'application d'une composition cosmétique telle que définie à l'une des revendications précédentes, sur les cheveux et/ou le cuir chevelu humains.

26. Utilisation cosmétique d'au moins copolymère bloc exclusivement constitué de blocs hydrophiles tel que défini dans l'une quelconque des revendications précédentes comme agent pour renforcer l'activité antipelliculaire d'une composition notamment cosmétique contenant un milieu physiologiquement acceptable et au moins un agent antifongique choisi parmi les sels de pyridinethione, les dérivés de 1-hydroxy-2-pyrrolidone, le 2-2'-dithio-bis-(pyridine-N-oxide), les sulfures de sélénium et leurs mélanges.
